Europäisches Patentamt

European Patent Office

Office européen des brevets

(19)

(11) **EP 1 380 281 A1**

(12) **DEMANDE DE BREVET EUROPEEN**

(43) Date de publication:
**14.01.2004 Bulletin 2004/03**

(51) Int Cl.⁷: **A61K 7/043**

(21) Numéro de dépôt: **03291518.3**

(22) Date de dépôt: **20.06.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **08.07.2002 FR 0208556**

(71) Demandeur: **L'OREAL**
**75008 Paris (FR)**

(72) Inventeurs:
• **Vicic, Marco**
**94360 Brys/Marne (FR)**
• **Cazeneuve, Colette**
**75007 Paris (FR)**
• **Mougin, Nathalie**
**75011 Paris (FR)**

(74) Mandataire: **Boulard, Denis**
**L'OREAL - D.I.P.I.**
**25-29 Quai Aulagnier**
**92600 Asnières (FR)**

(54) **Vernis à ongles**

(57) L'invention a pour objet une composition de vernis à ongles comprenant, dans un milieu cosmétiquement acceptable, un polymère filmogène, le vernis étant apte à former un film présentant une vitesse de perte de masse inférieure à 0,5 mg/minute lorsque le film est soumis à une d'abrasion effectuée avec l'abrasimètre TABER à 23 °C.

EP 1 380 281 A1

# Description

La présente invention a pour objet un vernis à ongles comprenant un polymère filmogène présentant de bonnes propriétés de tenue. L'invention a également pour objet un procédé de maquillage ou de soin des ongles.

La composition de vernis à ongles peut être employée comme base pour vernis, comme produit de maquillage des ongles, comme composition de finition, encore appelée "top-coat" en terminologie anglosaxonne, à appliquer sur le produit de maquillage des ongles ou bien encore comme produit de soin cosmétique des ongles. Ces compositions peuvent s'appliquer sur les ongles d'êtres humains ou bien encore sur des faux ongles.

Les compositions de vernis à ongles comprennent de façon connue un polymère filmogène dans un milieu solvant organique ou un milieu aqueux. Le vernis forme après séchage un film coloré ou incolore sur les ongles et permet ainsi d'embellir ou de protéger les ongles contre les agressions extérieurs tels que les frottements, les rayures. Or fréquemment, les vernis à ongles présentent une mauvaise tenue dans le temps : le film se détériore , notamment par écaillage ou décollement, au bout d'un ou deux jours. Une telle détérioration se produit souvent à l'extrémité de l'ongle. Lorsque le vernis est endommagé, l'utilisatrice doit alors retirer le vernis abimé puis procéder à une nouvelle application du vernis. L'utilisatrice peut également retoucher le vernis endommagé en appliquant partiellement du vernis mais ce type de retouche ne conduit pas à un maquillage parfaitement esthétique. Si l'utilisatrice ne fait rien, le vernis endommagé nuit à l'aspect estéthique du maquillage et n'assure pas une bonne protection de l'ongle.

D'autres vernis à ongles comme les vernis facilement pelable ou les vernis démaquillable à l'eau ne confèrent pas non plus une bonne tenue dans le temps.

Un besoin existe donc de disposer de vernis à ongles permettant d'obtenir un film déposé sur les ongles présentant une tenue dans le temps satisfaisante, sans présenter de défaut inesthétique.

Le but de la présente invention est donc de proposer une composition de vernis à ongles présentant de bonnes propriétés de tenue dans le temps, en particulier une bonne résistance aux frottements, à l'eau, à l'écaillage.

Les inventeurs ont découvert qu'un vernis à ongles présentant une faible vitesse de perte de masse pendant une abrasion permettait d'obtenir un vernis ayant une bonne tenue dans le temps.

De façon plus précise, l'invention a pour objet une composition de vernis à ongles comprenant, dans un milieu cosmétiquement acceptable, un polymère filmogène et un solvant organique, le vernis étant apte à former un film présentant une vitesse de perte de masse inférieure à 0,5 mg/minute lorsque le film est soumis à une d'abrasion effectuée avec l'abrasimètre TABER à 23 °C.

L'invention a aussi pour objet un procédé cosmétique de maquillage ou de soin non thérapeutique des ongles comprenant l'application sur les ongles d'au moins une couche de la composition de vernis à ongles telle que définie précédemment.

Dans la présente demande, on entend par "polymère filmogène", un polymère apte à former à lui seul, ou en présence d'agent auxiliiare de filmification, un film continu et adhérent sur l'ongle, à une température allant de 20 °C à 30 °C.

La vitesse de perte de masse du film de vernis à ongles est mesurée selon le protocole décrit ci-après.

On dépose une couche de la composition sur un support métallique (acier) pour obtenir après séchage pendant 24 heures à 23±2 °C et à 55±5% d'humidité relative, un film ayant une épaisseur d'environ 100μm (après séchage). Le film est soumis à une abrasion pendant 60 minutes à l'aide de l'abrasimètre TABER (référence 5130 Abraser) muni de deux roues abrasives vendue sous la dénomination CS10F par la société Taber Industries et en appliquant sur chaque roue une force de 2,5 N. Pendant l'opération d'abrasion, on mesure la vitesse de la perte de masse de film sur la période de 60 minutes.

Le film de vernis à ongles selon l'invention présente une vitesse de perte de masse inférieure à 0,5 mg/minute, de préférence inférieure à 0,2 mg/minute, et plus préférentiellement inférieure à 0,1 mg/minute.

Avantageusement, le film de vernis à ongles présente une perte de brillance, après 10 secondes d'abrasion avec l'abrasimètre TABER décrit précédemment, inférieure ou égale à 14 %, et de préférence inférieure ou égale à 12 %. La perte de brillance correspond au rapport (Bo- B)/Bo en %, dans lequel B représente la brillance du film après abrasion et Bo représente à la brillance du film avant abrasion La brillance du film est mesurée à l'aide d'un brillancemètre BYK-GARDNER à un angle de faisceau lumineux de 60°.

Selon un mode de réalisation selon l'invention, le polymère filmogène a en particulier au moins une température de transition vittreuse (Tg) allant de -150 °C à 0 °C, et de préférence allant de -80 °C à 0 °C.

Le polymère filmogène peut également avoir une température de transition vitreuse additionnelle supérieure à 0 °C et inférieure ou égale à 150 °C, de préférence allant de 10 °C à 100 °C.

La mesure de la température de transition vitreuse (Tg) du polymère est effectuée par DMTA (Dynamical and Mechanical Temperature Analysis ou Analyse dynamique et mécanique de température).

Pour mesurer la température de transition vitreuse (Tg) du polymère, on effectue des essais de viscoélasticimétrie avec un appareil DMTA de Polymer laboratories, sur un échantillon de film de polymère d'environ $150 \pm 50$ μm d'épaisseur, 5 mm de largeur et 10 mm de longueur, après séchage pendant 24 heures à 23 °C et à 50-55 % d'humidité relative. On impose à cet échantillon une sollicitation de traction. L'échantillon subit une force statique de 0,01 N à laquelle se superpose un déplacement sinusoïdal de ± 8 µm à la fréquence de 1 Hz. On travaille ainsi dans le domaine linéaire, sous de faibles niveaux de déformation. Cette sollicitation de traction est effectuée sur l'échantillon à des températures variant de -150 °C à + 220 °C, avec une variation de température de 3 °C par minute.

On mesure alors le module complexe E* = E' + iE" du polymère testé en fonction de la température.

De ces mesures, on déduit les modules dynamiques E' de conservation et E" de perte, ainsi que le pouvoir amortissant : tgδ = E"/E'.

Puis on trace la courbe des valeurs de tgδ en fonction de la température ; cette courbe présente au moins un pic. La température de transition vitreuse Tg du polymère correspond à la température à laquelle se situe le sommet de ce pic.

Lorsque la courbe présente au moins 2 pics (dans ce cas, le polymère présente au moins 2 Tg), on prend comme valeur de Tg du polymère testé la température pour laquelle la courbe présente le pic de plus forte amplitude (c'est à dire correspondant à la plus grande valeur de tgδ ; on ne considère dans ce cas que la Tg « majoritaire » comme valeur de Tg du polymère testé).

Selon un mode de réalisation de l'invention, le vernis à ongles selon l'invention est apte à former un film ayant un module d'Young allant de 10 à 200 MPa, de préférence allant de 10 à 100 MPa, et plus préférentiellement allant de 10 à 50 MPa.

On mesure les propriétés mécaniques du film de vernis à ongles en traction monotone selon la norme ASTM Standards, volume 06.01 D 2370-92 'Standard Test Method for Tensile Properties of Organic Coatings' . On découpe une éprouvette dans un film libre ayant une épaisseur de 150 ± 50µm obtenu après 48 heures de séchage à 23 ± 2 °C et 55 ± 5 % d'humidité relative d'une couche de vernis à ongles déposée sur une matrice en téflon. L'éprouvette est de forme haltère, de longueur utile 33 mm et de largeur utile 6 mm. La section (s) de l'éprouvette est alors définie comme : s=largeur x épaisseur (mm$^2$), cette section (s) sera utilisée pour le calcul de la contrainte .

Les essais sont réalisés sur un appareil de traction équipé d'un extensomètre optique pour la mesure du déplacement et commercialisé sous l'appellation Zwick Z010. Les mesures sont réalisées dans les mêmes conditions de température et d'humidité que pour le séchage,c'est-à-dire à une température de 23 ± 2 °C et une humidité relative de 55 ± 5 %. Les éprouvettes sont étirées à une vitesse de déplacement de 50 mm/mn.

On impose donc une vitesse de déplacement et on mesure simultanément la longueur (L) de l'éprouvette et la force (f) nécessaire pour imposer cette longueur.

La longueur (L) est mesurée avec un extensomètre optique à l'aide de pastilles adhésives placées sur l'éprouvette haltère. La distance initiale entre ces 2 pastilles définie la longueur utile Lo utilisée pour calculer la déformation ε = (L/Lo)×100 exprimée en %.

Il est ainsi obtenu une courbe contrainte σ ( = f/s) en fonction de la déformation ε, l'essai étant conduit jusqu'à rupture de l'éprouvette.

Le module d'Young (module d'élasticité), exprimé en MPa, correspond à la pente de la courbe σ = f(ε) , considérée dans la partie linéaire de la courbe (début de l'essai).

Avantageusement, le vernis à ongles selon l'invention est apte à former un film ayant une énergie à la rupture $W_r$ allant de $220 \times 10^5$ à $1000 \times 10^5$ J/m$^3$, et de préférence allant de $240 \times 10^5$ à $1000 \times 10^5$ J/m$^3$. L'énergie à la rupture $W_r$ exprimée en J/m$^3$ correspond à l'aire sous la courbe de la contrainte en fonction de la déformation ; l'aire est définie par la relation :

$$Wr = ((\int_0^{l\,\max} fdl)\,/(s.Lo)$$

dans laquelle :

- f désigne la force appliquée sur l'éprouvette de film ;
- s désigne la section de l'éprouvette de film ;
- Lo désigne la longueur initiale de l'éprouvette de film avant sa déformation
- lmax est la longueur maximale de l'éprouvette à la fin de la phase d'étirement.

Selon un mode particulier de réalisation de l'invention, le vernis à ongles selon l'invention est apte à former un film ayant une déformation à la rupture $ε_r$ allant de 300 à 2000 %, et de préférence allant de 500 à 2000 %. La déformation à la rupture $ε_r$ est la déformation maximale de l'échantillon avant le point de rupture (en %).

Avantageusement, le polymère filmogène est insoluble dans l'eau à 25 °C, c'est-à-dire qu'il est soluble à moins de 1 % en poids dans l'eau à 25 °C (solubilité inférieure à 1 % en poids). Le polymère est de préférence soluble dans les solvants organiques, comme l'acétate d'éthyle ou l'acétate de méthyle (solubilité supérieure à 90 % en poids à 25 °C).

Le polymère filmogène a notamment un poids moléculaire moyen en nombre inférieur ou égal à 300000, et de préférence allant de 10000 à 150000.

Le polymère filmogène permettant d'obtenir les propriétés de résistance à l'abrasion du vernis peut être choisi parmi les polymères radicalaires, les polycondensats, et de préférence parmi les polycondensats. Le polymère est en particulier non réticulé.

Le polymère peut être en particulier un polycondensat, et notamment choisi parmi polyuréthanes, les polyurées, les polyurée-uréthanes, et leurs mélanges.

Le polycondensat est de préférence choisi parmi ceux formés par polycondensation :

- d'au moins un diisocyanate choisi parmi les diisocyanates d'alkyle linéaire ou ramifié en $C_1$-$C_{12}$, de préférence en $C_1$-$C_6$, les diisocyanates de cycloalkyle en $C_4$-$C_{20}$ , les diisocyanates d'aryle en $C_6$-$C_{20}$;
- d'au moins un prépolymère comprenant au moins 2 fonctions comportant un hydrogène labile, notamment un diol ou une diamine primaire ou secondaire, ayant une masse moléculaire en nombre allant de 500 à 50000, de préférence allant de 1000 à 3000 et préférentiellement allant de 500 à 8000 ;
- d'au moins un coupleur comprenant deux fonctions comportant un hydrogène labile, notamment un diol ou une diamine primaire ou secondaire ou un aminoalcool, ayant une masse moléculaire inférieure 500, notamment supérieur ou égal à 50 et inférieur à 500, et de préférence supérieur ou égal à 75 et inférieur à 500.

Le diisocyanate peut être par exemple le diisocyanate d'hexaméthylène, le diisocyanate d'isophorone, le diisocyanate de dicyclohexylméthane, le diisocyanate de toluène, le diisocyanate de diphénylméthane, le diisocyanate de dicyclohexylméthane, le diisocyanate de tétraméthylxylylène.

Le prépolymère décrit précédemment peut être par exemple un diol de polyoxyde de tétraméthylène ayant de 10 à 80 d'oxyde de tétraméthylène; un polydiméthylsiloxane comprenant des groupements terminaux de type alkylène($C_2$-$C_8$)amino alkyl ($C_2$-$C_8$) ou ou de type ω-hydroxyalkyle en $C_2$-$C_8$; un polybutadiène hydrogéné ayant des groupes hydroxyle terminaux.

Le coupleur est de préférence un diol, notamment un diol ayant de 4 à 12 atomes de carbone, et préférentiellement ayant de 4 à 8 atomes de carbone. Comme coupleur, on peut citer le butanediol, le néopentylglycol, l'aminoéthanol, le propylèneglycol, l'éthylèneglycol, le diéthylèneglycol, le triéthylèneglycol, le cyclohexane diméthanol.

Le prépolymère est préférentiellement non hydrosoluble (le prépolymère a une solubilité dans l'eau inférieure à 1 % en poids, à 25 °C).

Avantageusement, le prépolymère et le coupleur décrits précédemment sont présents dans le polyuréthane en une teneur telle que le rapport molaire prépolymère / coupleur va de 1 à 1/5 et le rapport molaire (prépolymère + coupleur) / diisocyanate va de 0,9 à 1,1.

Lorsque le rapport pondéral (prépolymère + coupleur) / diisocyanate est inférieur à 1, les groupes isocyanates libres sont bloqués par réaction avec un composé comprenant un hydrogène labile comme l'éthanol.

De préférence, le polymère est tel que le prépolymère, le diisocyanate et le coupleur sont présents dans le polymère selon un rapport molaire :

Prépolymère : 1
Diisocyanate : 2 à 6
Coupleur : 1 à 5.

Lorsque le rapport pondéral (prépolymère + coupleur) / diisocyanate est inférieur à 1, les groupes isocyanates libres sont bloqués par réaction avec un composé comprenant un hydrogène labile comme l'éthanol.

Le polymère filmogène peut être présent dans la composition de vernis à ongles en une teneur allant de 0,1% à 60% en poids, par rapport au poids total de la composition, et de préférence allant de 0,1% à 40% en poids.

Le vernis à ongles selon l'invention peut comprendre en outre un polymère filmogène additionnel, appelée couramment résine, comme les résines sulfonamides, les résines alkyde, les esters de cellulose tel que l'acétobutyrate de cellulose, l'acétate de cellulose, l'acétopropionate de cellulose.

Le polymère filmogène additionnel peut être présent en une teneur allant de 0,01 % à 50 % en poids, par rapport au poids total de la composition, et de préférence allant de 1 % à 30 % en poids.

La composition de vernis à ongles selon l'invention peut comprendre un agent auxiliaire de filmification pour améliorer les propriétés filmogènes du vernis.

L'agent auxiliaire de filmification peut être choisi parmi tous les composés connus de l'homme du métier comme étant susceptibles de remplir la fonction recherchée, et être notamment choisi parmi les agents plastifiants.

Comme exemple d'agent plastifiant, on peut citer :

les citrates tels que le citrate de triéthyle, le citrate de tributyle, l'acétylcitrate de triéthyle, l'acétylcitrate de tributyle, l'acétylcitrate de triéthyl-2 hexyle
les phtalates tels que le phtalate de diéthyle, le phtalate de dibutyle, le phtalate de dioctyle, le phtalate de dipentyle, le phtalate de diméthoxyéthyle,
le phosphate de tricrésyle, le benzoate de benzyle, le phosphate de tributyle, l'acétylricinoléate de butyle, l'acétylricinoléate de glycéryle, le glycolate de butyle, le phosphate de tributoxyéthyle, le phosphate de triphényle, le tartrate de dibutyle, le camphre, le triacétate de glycérol, le N-éthyl-o,p-toluènesulfonamide, et leurs mélanges.

L'agent plastifiant peut être présent dans la composition en une teneur allant de 0,01 % à 10 % en poids, par rapport au poids total de la composition, et de préférence allant de 0,1 % à 5 % en poids. Avantageusement, le plastifiant est présent dans la composition selon un rapport pondéral polymère filmogène / plastifiant allant de 1,5 à 3.

La composition selon l'invention peut comprendre un milieu aqueux ou un milieu solvant organique, et de préférence un milieu solvant organique. Elle peut être notamment anhydre.

Le milieu aqueux de la composition peut être constitué essentiellement d'eau. La teneur en eau dans la composition peut aller de 10 % à 95 % en poids, par rapport au poids total de la composition, de préférence de 40 % à 90 % en poids, et mieux de 60 % à 85 % en poids.

La composition peut comprendre également un solvant miscible à l'eau comme les monoalcools inférieurs ayant de 1 à 5 atomes de carbone, les glycols ayant de 2 à 8 atomes de carbone, les cétones en $C_3$-$C_4$, les aldéhydes en $C_2$-$C_4$, notamment en une teneur pouvant aller de 0,1 % à 15 % en poids, par rapport au poids total de la composition.

Lorsque la composition comprend un milieu aqueux, le polymère filmogène est présent sous formes de particules solides dispersées dans le mileu aqueux. Une telle dispersion est connue sous le nom de latex ou de pseudolatex et peut être préparée selon les techniques bien connues de l'homme du métier.

Le milieu solvant organique de la composition peut comprendre un ou plusieurs solvants organiques choisis parmi :

- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le n-propanol, le n-butanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, l'isododécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde
- leurs mélanges.

La teneur en solvant organique dans la composition peut aller de 10 % à 95 % en poids, par rapport au poids total de la composition, de préférence de 40 % à 90 % en poids, et mieux de 60 % à 85 % en poids.

La composition peut comprendre un agent épaississant, notamment pour conférer à la composition une consistance permettant une bonne application de la composition sur les ongles. L'agent épaississant est plus particulièrement un épaississant de solvant organique et peut être choisi parmi les silices hydrophobes, telles que celles dérites dans le document EP-A-898960, et par exemple commercialisées sous les références "AEROSIL R812®" par la société Degussa, "CAB-O-SIL TS-530®", "CAB-O-SIL TS-610®", "CAB-O-SIL TS-720®"par la société Cabot, "AEROSIL R972®", "AEROSIL R974®" par la société Degussa ; les argiles telles que la montmorillonite, l'hectorite stéralkonium, la bentonite stéralkonium ; les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958, et par exemple commercialisés sous les dénominations "N-HANCE-AG 200®" et "N-HANCE AG 50®" par la société Aqualon.

L'agent épaississant peut être présent dans la composition selon l'invention en une teneur allant de 0,05 % à 10 % en poids, par rapport au poids total de la composition, et de préférence allant de 0,1 % à 3 % en poids.

La composition peut comprendre également une matière colorante qui peut être choisie parmi les colorants liposolubles, les pigments, les nacres, les paillettes.

Les colorants liposolubles peuvent être par exemple le rouge Soudan, le DC Red 17, le DC Green 6, le β-carotène, l'huile de soja, le brun Soudan, le DC Yellow 11, le DC Violet 2, le DC orange 5, le jaune quinoléine. Ils sont généralement présents dans la composition en une teneur pouvant aller de 0,01 % à 6 % en poids, par rapport au poids total de la composition, de préférence allant de 0,01 % à 3 % en poids.

Les pigments, les nacres et les pailettes peuvent être présents dans la composition, notamment la composition de base et/ou de surface, en une teneur allant de 0,01 % à 25 % en poids, par rapport au poids de la composition, de préférence de 0,01 % à 15 % en poids. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium. Parmi les nacres, on peut citer le mica recouvert d'oxyde de titane, d'oxyde de fer, de pigment naturel ou d'oxychlorure de bismuth tel que le mica titane coloré.

La composition selon l'invention, notamment la composition de base et/ou de surface, peut en outre comprendre tout additif cosmétique connu de l'homme du métier comme étant susceptible d'être incorporé dans une telle composition, tels que les charges, les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants. Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

L'invention est illustrée plus en détail dans les exemples suivants.

**Exemple 1 :**

a) On a préparé le polyuréthane suivant :

Dans un réacteur de 500 ml équipé d'une agitation centrale, d'un réfrigérant, on introduit sous azote 48,75 g (1 mole) de polyoxyde de tétraméthylène de poids moléculaire 1400, 12,55 g (4 moles) de butanediol et 33,3 g de méthyléthylcétone.

On agite à température ambiante puis on porte le milieu réactionnel au reflux du solvant pendant 15 minutes. On ajoute ensuite goutte à goutte pendnat 30 minutes 39,9 g (5,15 moles) de diisocyanate d'isophorone. On laisse réagir pendant 1 heure puis on ajoute un mélange de 5 ml de méthyléthylcétone et de 0,1 g de dibutyl dilaurate d'étain. On ajoute 60 g de méthyléthylcétone, on laisse réagir pendant 1heure 10 minutes et on ajoute encore 60 g de méthyléthylcétone. Puis on ajoute 20 g d'éthanol pour neutraliser les fonctions isocyanates résiduelles.

Le polymère synthétisé est précipité dans de l'éther de pétrole puis séché à 40 °C sous vide en présence d'anhydride phosphorique.

Le polymère présente les caractéristiques suivantes :

Poids moléculaire moyen en nombre : 62426 g/mole

Indice de polydispersité = 1,6

Température de transition vitreuse : - 50 °C et + 60 °C.

On a préparé un vernis à ongles ayant la composition suivante :

On a dissout 25 g du polymère obtenu dans 70 g d'un mélange acétate de butyle/acétate d'éthyle 70/30 en poids.

La solution obtenue est ensuite appliquée sur les ongles. On obtient après séchage un film de vernis qui a les caractéristiques suivantes, mesurées selon les protocoles décrits précedemment dans la description :

Vitesse de perte de masse lorsque le film est soumis à une d'abrasion effectuée avec l'abrasimètre TABER à 23 °C : < 0,1 mg/minute Perte de brillance après 1 heure : 3 ±1 % Module d'Young = 45 ± 5 MPa Energie à la rupture = $250 \times 10^5$ J/m$^3$ Déformation à la rupture = 500 ± 50 %

Le vernis après application sur l'ongle forme un film présentant une bonne résistance à l'usure.

**Revendications**

1. Vernis à ongles comprenant, dans un milieu cosmétiquement acceptable, un polymère filmogène, le vernis étant apte à former un film présentant une vitesse de perte de masse inférieure à 0,5 mg/minute lorsque le film est soumis à une d'abrasion effectuée avec l'abrasimètre TABER à 23 °C.

2. Vernis à ongles selon la revendication 1, **caractérisé par le fait que** le film de vernis à ongles présente une vitesse de perte de masse inférieure à 0,2 mg/minute, et de préférence inférieure à 0,1 mg/minute.

3. Vernis à ongles selon l'une quelconque des revendications précédentes, **caractérisé par le fait que** le polymère à au moins une température de transition vitreuse allant de -150 °C à 0 °C.

4. Vernis à ongles selon l'une quelconque des revendications précédentes, **caractérisé par le fait que** le polymère a au moins une température de transition vitreuse allant de -80 °C à 0 °C.

5. Vernis à ongles selmon l'une quelconque des revendications précédentes, **caractérisé par le fait que** le polymère filmogène a une température de transition vitreuse additionnelle supérieure à 0 °C et inférieure ou égale à 150 °C, de préférence allant de 10 °C à 100 °C.

6. Vernis à ongles selon l'une quelconque des revendications précédentes, **caractérisé par le fait qu'**il est apte à former un film ayant un module d'Young allant de 10 à 200 MPa.

7. Vernis à ongles selon la revendication 6, **caractérisé par le fait que** le film a un module allant de 10 à 100 MPa , et de préférence allant de 10 à 50 MPa.

8. Vernis à ongles selon l'une quelconque des revendications précédentes, **caractérisé par le fait qu'**il est apte à former un film ayant une énergie à la rupture allant de $220 \times 10^5$ à $1000 \times 10^5$ J/m$^3$.

9. Vernis à ongles selon la revendication 8, **caracté-**

**risé par le fait que** le film a une énergie à la rupture allant de 240 × 10⁵ à 1000 × 10⁵ J/m³.

10. Vernis à ongles selon l'une quelconque des revendications précédentes, **caractérisé par le fait qu'il** est apte à former un film ayant une déformation à la rupture allant de 300 à 2000 %.

11. Vernis à ongles selon la revendication 10, **caractérisé par le fait que** le film a une déformation à la rupture allant de 500 à 2000 %.

12. Vernis à ongles selon l'une quelconque des revendications précédentes, **caractérisé par le fait qu'il** est apte à former un film ayant une perte de brillance, après 10 secondes d'abrasion avec l'abrasimètre TABER et la brillance étant mesurée 1 heure après l'abrasion effectuée, inférieure ou égale à 14 %, de préférence inférieure ou égale à 10 %, et préférentiellement inférieur à 6 %.

13. Vernis à ongles selon l'une quelconque des revendications précédentes, **caractérisé par le fait que** le polymère filmogène est choisi parmi les polyuréthanes, les polyurées, les polyurée-uréthanes, et leurs mélanges.

14. Vernis à ongles selon l'une quelconque des revendications 1 à 13, **caractérisé par le fait que** le polymère filmogène est un polycondensat formé par polycondensation :

    - d'au moins un diisocyanate choisi parmi les diisocyanates d'alkyle linéaire ou ramifié en C₁-C₁₂, les diisocyanates de cycloalkyle en C₄-C₂₀ , les diisocyanates d'aryle en C₆-C₂₀ ;
    - d'au moins un prépolymère comprenant au moins 2 fonctions comportant un hydrogène labile, notamment un diol ou une diamine primaire ou secondaire, ayant une masse moléculaire en nombre allant de 500 à 50000, de préférence allant de 1000 à 3000 et préférentiellement allant de 500 à 8000 ;
    - d'au moins un coupleur comprenant deux fonctions comportant un hydrogène labile, notamment un diol ou une diamine primaire ou secondaire ou un aminoalcool, ayant une masse moléculaire inférieure 500, notamment supérieur ou égal à 50 et inférieur à 500, et de préférence supérieur ou égal à 75 et inférieur à 500.

15. Vernis à ongles selon la revendication précédente, **caractérisé par le fait que** le diisocyanate est choisi parmi le diisocyanate d'hexaméthylène, le diisocyanate d'isophorone, le diisocyanate de dicyclohexylméthane, le diisocyanate de toluène, le diisocyanate de diphénylméthane, le diisocyanate de dicyclohexylméthane, le diisocyanate de tétraméthylxylylène.

16. Vernis à ongles selon la revendication 14 ou 15, **caractérisé par le fait que** le prépolymère est choisi parmi un diol de polyoxyde de tétraméthylène ayant de 10 à 80 motifs d'oxyde de tétraméthylène ; un polydiméthylsiloxane comprenant des groupements terminaux de type alkylène (C₂-C₈)amino alkyl (C₂-C₈) ou ou de type ω-hydroxyalkyle en C₂-C₈ ; un polybutadiène hydrogéné ayant des groupes hydroxyle terminaux.

17. Vernis à ongles selon l'une quelconque des revendications 14 à 16, **caractérisé par le fait que** le prépolymère est non hydrosoluble.

18. Vernis à ongles selon l'une quelconque des revendications 14 à 17, **caractérisé par le fait que** le coupleur est choisi parmi le butanediol, le néopentylglycol, l'aminoéthanol, le propylèneglycol, l'éthylèneglycol, le diéthylèneglycol, le triéthylèneglycol, le cyclohexane diméthanol.

19. Vernis à ongles selon l'une quelconque des revendications 14 à 18, **caractérisé par le fait que** le prépolymère et le coupleur sont présents dans le polyuréthane en une teneur telle que le rapport molaire prépolymère / coupleur va de 1 à 1/5 et le rapport molaire (prépolymère + coupleur) / diisocyanate va de 0,9 à 1,1.

20. Vernis à ongles selon l'une quelconque des revendications 14 à 18, **caractérisé par le fait que** le polymère est tel que le prépolymère, le diisocyanate et le coupleur sont présents dans le polymère selon un rapport molaire :

    Prépolymère : 1
    Diisocyanate : 2 à 6
    Coupleur : 1 à 5.

21. Vernis à ongles selon l'une quelconque des revendications 14 à 20, **caractérisé par le fait que** lorsque le rapport pondéral (prépolymère + coupleur) / diisocyanate est inférieur à 1, les groupes isocyanates libres sont bloqués par réaction avec un composé comprenant un hydrogène labile comme l'éthanol.

22. Vernis à ongles selon l'une quelconque des revendications précédentes, **caractérisé par le fait que** le polymère filmogène a un poids moléculaire moyen en nombre inférieur ou égal à 300000, et de préférence allant de 10000 à 150000.

23. Vernis à ongles selon l'une quelconque des revendications précédentes, **caractérisé par le fait que** le polymère filmogène est présent en une teneur allant de 0,1 % à 60% en poids, par rapport au poids total de la composition, et de préférence allant de 0.1% à 40% en poids.

**24.** Vernis à ongles selon l'une quelconque des revendications précédentes, **caractérisé par le fait qu'**il comprend un polymère filmogène additionnel choisi parmi les résines sulfonamides, les résines alkyde, les esters de cellulose.

**25.** Vernis à ongles selon l'une quelconque des revendications précédentes, **caractérisé par le fait qu'**il comprend un agent plastifiant.

**26.** Vernis à ongles selon l'une quelconque des revendications précédentes, **caractérisé par le fait qu'**il comprend un milieu solvant organique.

**27.** Vernis à ongles selon la revendication précédente, **caractérisé par le fait qu'**il est anhydre.

**28.** Vernis à ongles selon l'une quelconque des revendications 1 à 26, **caractérisé par le fait qu'**il comprend un milieu aqueux.

**29.** Vernis à ongles selon l'une quelconque des revendications précédentes, **caractérisé par le fait qu'**il comprend un additif cosmétique choisi parmi les agents épaississants, les matières colorantes, les charges, les agents d'étalement, les agents mouillants, les agents dispersants, les anti-mousses, les conservateurs, les filtres UV, les actifs, les tensioactifs, les agents hydratants, les parfums, les neutralisants, les stabilisants, les antioxydants.

**30.** Procédé cosmétique de maquillage ou de soin non thérapeutique des ongles comprenant l'application sur les ongles d'au moins une couche de vernis à ongles selon l'une quelconque des revendications précédentes.

EP 1 380 281 A1

**Office européen des brevets**

# RAPPORT PARTIEL
# DE RECHERCHE EUROPEENNE

qui selon la règle 45 de la Convention sur le brevet européen est consideré, aux fins de la procédure ultérieure, comme le rapport de la recherche européenne

Numéro de la demande

EP 03 29 1518

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | WO 02 39962 A (AVON PRODUCTS, INC.) 23 mai 2002 (2002-05-23) * le document en entier * --- | 1-27,29, 30 | A61K7/043 |
| X | US 5 681 550 A (M. RUBINO) 28 octobre 1997 (1997-10-28) * le document en entier * --- | 1-26, 28-30 | |
| X | EP 0 658 609 A (L'OREAL) 21 juin 1995 (1995-06-21) * revendications 1,19; exemples 1-6 * --- | 1-30 | |
| P,X | FR 2 819 176 A (L'OREAL) 12 juillet 2002 (2002-07-12) * revendications 1,34; exemple 1 * --- | 1-23,25, 26,28-30 | |
| X | FR 2 814 674 A (L'OREAL) 5 avril 2002 (2002-04-05) * revendications 1,19; exemple 8 * --- | 1-13 | |
| | -/-- | | |

**DOMAINES TECHNIQUES RECHERCHES (Int.Cl.7)**

A61K
A61Q

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| LA HAYE | 2 octobre 2003 | Glikman, J-F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C08)

9

**Office européen**

**des brevets**

**RECHERCHE INCOMPLETE**
**FEUILLE SUPPLEMENTAIRE C**

Numéro de la demande

EP 03 29 1518

Revendications ayant fait
l'objet de recherches complètes:
        13-30

Revendications ayant fait
l'objet de recherches incomplètes:
        1-12

Raison pour la limitation de la recherche:

Les revendications 1-12 présentes ont trait à une composition définie
(entre autres) au moyen des paramètres suivants:
P1: une abrasion avec l'abrasimètre Taber
P2: une température de transition vitreuse
            P3: un module de Young
            P4: une énergie à la rupture
            P5: une déformation à la rupture
            P6: une perte de brillance après abrasion

L'utilisation de ces paramètres est considérée , dans le présent
contexte, comme menant à un manque de clarté au sens de l'Article 84 CBE.
Il est impossible de comparer les paramètres que le déposant a choisi
d'utiliser avec ce qui est révélé dans l'état de la technique. Le manque
de clarté qui en découle est tel q'une recherche significative complète
est impossible.
 De plus les revendications 1-12 semblent manquer de support dans la
mesure où l'homme du métier de la cosmétique serait mis en difficulté
pour préparer des compositions selon ces revendications 1-12.
 Par conséquent, la recherche a été limitée aux compositions contenant
des polycondensats tels que définis à la p.7, lig.7-20 de la présente
demande et susceptibles de présenter une des caractéristiques énoncées
dans les présentes revendications 1-12.

**Office européen**

**des brevets**

**RAPPORT PARTIEL**

**DE RECHERCHE EUROPEENNE**

Numero de la demande

EP 03 29 1518

| | DOCUMENTS CONSIDERES COMME PERTINENTS | | CLASSEMENT DE LA DEMANDE (Int.Cl.7) |
|---|---|---|---|
| Catégorie | Citation du document avec indication, en cas de besoin, des parties pertinentes | Revendication concernée | |
| A | H.F. MARK ET AL.: "Encyclopedia of polymer science and engineering, edition 2, vol.13" 1988 , J. WILEY AND SONS , US XP002238035 156530 * page 294 * | 1-30 | |
| | --- | | |
| A | J.M. BUIST ET AL. ED.: "Advances in polyurethane technology" 1968 , MACLAREN AND SONS LTD , LONDON XP002238036 700014 * page 286 * * page 296 - page 297 * | 1-30 | |
| | ----- | | |

DOMAINES TECHNIQUES RECHERCHES (Int.Cl.7)

EPO FORM 1503 03.82 (P04C11)

**ANNEXE AU RAPPORT DE RECHERCHE EUROPEENNE
RELATIF A LA DEMANDE DE BREVET EUROPEEN NO.**

EP 03 29 1518

La présente annexe indique les membres de la famille de brevets relatifs aux documents brevets cités dans le rapport de
recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

02-10-2003

| Document brevet cité au rapport de recherche | | Date de publication | Membre(s) de la famille de brevet(s) | | Date de publication |
|---|---|---|---|---|---|
| WO 0239962 | A | 23-05-2002 | AU | 2567902 A | 27-05-2002 |
| | | | BR | 0107485 A | 03-09-2002 |
| | | | CA | 2394355 A1 | 23-05-2002 |
| | | | CN | 1392794 T | 22-01-2003 |
| | | | EP | 1333791 A1 | 13-08-2003 |
| | | | WO | 0239962 A1 | 23-05-2002 |
| US 5681550 | A | 28-10-1997 | AUCUN | | |
| EP 658609 | A | 21-06-1995 | FR | 2713649 A1 | 16-06-1995 |
| | | | AT | 210708 T | 15-12-2001 |
| | | | CA | 2137736 A1 | 16-06-1995 |
| | | | DE | 69429414 D1 | 24-01-2002 |
| | | | DE | 69429414 T2 | 06-06-2002 |
| | | | EP | 0658609 A1 | 21-06-1995 |
| | | | ES | 2169733 T3 | 16-07-2002 |
| | | | JP | 2682960 B2 | 26-11-1997 |
| | | | JP | 7207102 A | 08-08-1995 |
| | | | US | 5911973 A | 15-06-1999 |
| | | | US | 6113925 A | 05-09-2000 |
| FR 2819176 | A | 12-07-2002 | FR | 2819176 A1 | 12-07-2002 |
| FR 2814674 | A | 05-04-2002 | FR | 2814674 A1 | 05-04-2002 |

EPO FORM P0460

Pour tout renseignement concernant cette annexe : voir Journal Officiel de l'Office européen des brevets, No.12/82